(19) Europäisches Patentamt
European Patent Office
Office européen des brevets

(11) Veröffentlichungsnummer: **0 001 740**
**B1**

(12) **EUROPÄISCHE PATENTSCHRIFT**

(21) Anmeldenummer: **78100705.9**

(22) Anmeldetag: **18.08.78**

(51) Int. Cl.³: **C 07 C 69/67, C 07 C 69/73, C 07 C 67/30**

(54) **Verfahren zur Herstellung von 3-Halogenalkoxy- und 3-Hydroxyalkoxy-2-alkyl-propionsäureestern**

(30) Priorität: **31.10.77 CH 13188/7**

(43) Veröffentlichungstag der Anmeldung:
**16.05.79 Patentblatt 79/10**

(45) Bekanntmachung des Hinweises auf die Patenterteilung:
**03.09.80 Patentblatt 80/18**

(84) Benannte Vertragsstaaten:
**DE FR GB**

(56) Entgegenhaltungen:
**FR - A - 2 128 410**

(73) Patentinhaber: **Bracco Industria Chimica Società per Azioni**
**Via E. Folli, 50**
**I - 20134 Milano (IT)**

(72) Erinder: **Felder, Ernst, Prof. Dr.**
**Via Ceresio 49**
**CH - 6826 Riva S. Vitale (CH)**
**Pitrè, Davide, Prof. Dr.**
**Via Brocchi 24**
**Milano (IT)**

(74) Vertreter: **Zutter, Hans Johann Niklaus**
**EPROVA Aktiengesellschaft im Laternenacker 5**
**CH - 8200 Schauffhausen (CH)**

**EP 0 001 740 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 3-Halogenalkoxy- und 3-Hydroxyalkoxy-2-alkyl-propionsäureestern

Die vorliegende Erfindung betrifft ein wesentlich verbessertes arbeitshygienisches Verfahren zur Herstellung von 3-Halogenalkoxy-2-alkyl-propionsäureestern und von 3-Hydroxyalkoxy-2-alkyl-propionsäureestern der allgemeinen Formel

Alkyl
|
X–Alkylen–O–$CH_2$–CH–COOR   I,

worin X Chlor, Brom oder eine Hydroxy-Gruppe, Alkylen einen Alkylen-Rest mit 2 bis 4 Kohlenstoffatomen und Alkyl einen Alkyl-Rest mit 1 bis 4 Kohlenstoffatomen und R einen Alkyl-Rest mit 1 bis 3 Kohlenstoffatomen darstellt.

3-Halogenalkoxy-2-alkyl-propionsäureester oder 3-Hydroxyalkoxy-2-alkyl-propionsäureester sind unerlässliche Zwischenprodukte für die Herstellung von bestimmten Röntgenkontrastmitteln, wie des oralen Gallenkontrastmittels bestehend aus 2-[2-(3-Acetamino-2,4,6-trijodphenoxy)-äthoxy]-methyl-buttersäue, die unter der markenfreien Bezeichnung IOPRONIC ACID bekannt ist. Vergl. dazu E. Felder et al, Il Farmaco *31* (1976) (Ed. Prat.) 283, 383, 347, 437, 516, 529, 540 (Ed. Sci.), 349, 426, 755.

Ein Verfahren zur Herstellung von 3-Halogenalkoxy-2-alkylpropionsäureestern ist bereits beschrieben worden. Nach Felder et al [deutsche Patentschrift 2.128.902] werden dazu Halogenalkoxy-methylchloride mit Natrium Alkyl-malonsäuredialkylestern zu den entsprechenden 2-(Halogenalkoxy)-2-alkyl-malonsäuredialkylestern umgesetzt, anschliessend selektiv zu den 2-(Halogenalkoxy)-2-alkyl-malonsäuremonoalkylestern verseift und diese zu den gewünschten 3-Halogenalkoxy-2-alkyl-propionsäureestern decarboxyliert.

Dieses Verfahren ist aufwendig und nicht sehr ergiebig. Es stützt sich ausserdem einerseitz auf teures Ausgangsmaterial — Alkylmalonsäuredialkylester — und anderseits auf arbeitshygienisch sehr bedenkliche Halogenalkoxy-methylchloride als Zwischenprodukte.

Halogenalkoxy-methylchloride gehören zu der Gruppe der stark cancerogenen $\alpha$-Haloäther. Sie enthalten überdies stets Bis-(chloromethyl)äther BCME dessen Carcinogenität als erstem Vertreter dieser Gruppe erkannt wurde. L.D. Taylor et al, J. Physical. Chem. *78* (1974) 2696. $\alpha$-Haloäther sind starke Mutagene, die schon nach kurzer Exposition Haut-, Trachea- und Lungen-Krebs erzeugen: R.T. Drew, S. Laskin und M. Kuschner, Arch. Euviron Health *30* (1975) 61, 70, 73. Wegen der hohen Gefährlichkeit dieser Stoffe wurden zu ihrer Erknnung feinste analytische Methoden entwickelt, die deren Bestimmung noch in Konzentrationen von 1 bis 0,01 ppb [part per billion ($10^9$)] erlauben: J.C. Tou et al, J. Physical. Chem. *78* (1974) 1096, J.C. Tou et al, Analyt. Chem. *46* (1974), 1866, L.A. Shadoff et al, Analyt. Chem. *45* (1973), 2341, K.P. Evans et al. Analyt. Chem. *47* (1975), 821, R.A. Salomon et al, Analyt. Chem. *47* (1975) 955.

Durch sinngemässe Anwendung einer von Lapkin beschriebenen allgemeinen Verfahrensweise [Zhur. Organ. Khim *2*(3), 393 — 5 (1966); Chemical Abstracts *65*.8753c (1966)], konnte in der Umsetzung von Chloralkoxymethylchlorid mit 2-Brom-alkansäureestern in Gegenwart von Zink gemäss:

Alkyl
|
Halogen–Alkylen–O–$CH_2$Cl + Zn + Br–CH–COOAlkyl ⟶

Alkyl
|
Halogen–Alkylen–O–$CH_2$–CH–COOAlkyl

ein Herstellverfahren für die gewünschten Zwischenprodukte der Formel (I) gefunden werden, welches wesentlich rationeller ist als das ursprüngliche Verfahren.

Trotzdem ist auch dieses verbesserte Verfahren für eine Fabrikation ungeeignet. Es muss in Diäthyläther durchgeführt werden, benötigt teure 2-Bromalkansäureester als Ausgangsmaterial und stützt sich, wie das ursprüngliche Verfahren, auf die stark cancerogenen $\alpha$-Haloäther als Zwischenprodukte. Von der Verwendung dieser gefährlichen Verbindungen selbst im Labormassstab wird in der Literatur gewarnt. Ihre Verwendung in einem Fabrikationsverfahren muss, nach dem heutigen Stand des Wissens, völlig ausser Betracht fallen.

Es wurde nun ein überraschend einfaches, billiges und ergiebiges Verfahren gefunden, welches sich auf billige und arbeitshygienische Ausgangsmaterialien stützt.

Dieses erfindungsgemässe Verfahren ist dadurch gekennzeichnet, dass man einen 2-Formylalkansäureester, der in an sich bekannter Weise durch Formylierung eines Alkansäureesters der allgemeinen Formel

Alkyl
|
$CH_2$–COOR   II,

worin Alkyl einen Alkyl-Rest mit 1 bis 4 Kohlenstoffatomen und R einen Alkyl-Rest mit 1 bis 3 Kohlenstoffatomen darstellt, mit Kohlenmonoxyd unter geringem Ueberdruck in Gegenwart eines Alkalimetallalkoholates erhalten wird,

a) in der dabei unmittelbar gebildeten Form seines Alkalimetall-Enolates der Formel

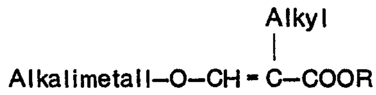

III,

direkt mit einem Halogen- oder Hydroxyalkylhalogenid oder mit einem Sulfonsäure-halogenalkyl- oder -hydroxyalkylester der allgemeinen Formel

X–Alkylen–Y  IV,

worin X Chlor, Brom oder eine Hydroxy-Gruppe, Alkylen einen zweiwertigen Alkylen-Rest mit 2 bis 4 Kohlenstoffatomen und Y Chlor, Brom oder Jod, oder eine Aryl- oder Alkylsulfonyloxy-Gruppe mit 1 bis 7 Kohlenstoffatomen bedeutet, in einem nicht wässrigen basischen Milieu zur Reaktion bringt oder

b) einen freigesetzten 2-Formylalkansäureester der Formel

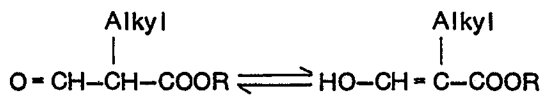

V,

mit einem Halogenalkanol oder Hydroxyalkanol der allgemeinen Formel

X–Alkylen–OH  VI,

worin X Chlor, Brom oder eine Hydroxy-Gruppe und Alkylen einen zweiwertigen Alkylen-Rest mit 2 bis 4 Kohlenstoffatomen bedeutet, in Gegenwart einer starken Säure und eines inerten organischen Lösungsmittels kondensiert und das Reaktionswasser laufend aus der Reaktionszone entfernt, wobei sowohl nach a) als auch nach b) der entsprechende 3-Halogenalkoxy- bzw. 3-Hydroxyalkoxy-2-alkylacrylsäureester der allgemeinen Formel

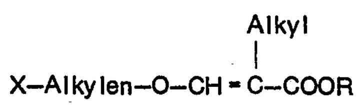

VII,

entsteht und dass man diesen anschliessend durch Hydrierung in den gewünschten 3-Halogenalkoxy- bzw. 3-Hydroxyalkoxy-2-alkyl-propionsäureester überführt.

Das erfindungsgemässe Verfahren kann etwa durch folgen- des Reaktionsschema illustriert werden.

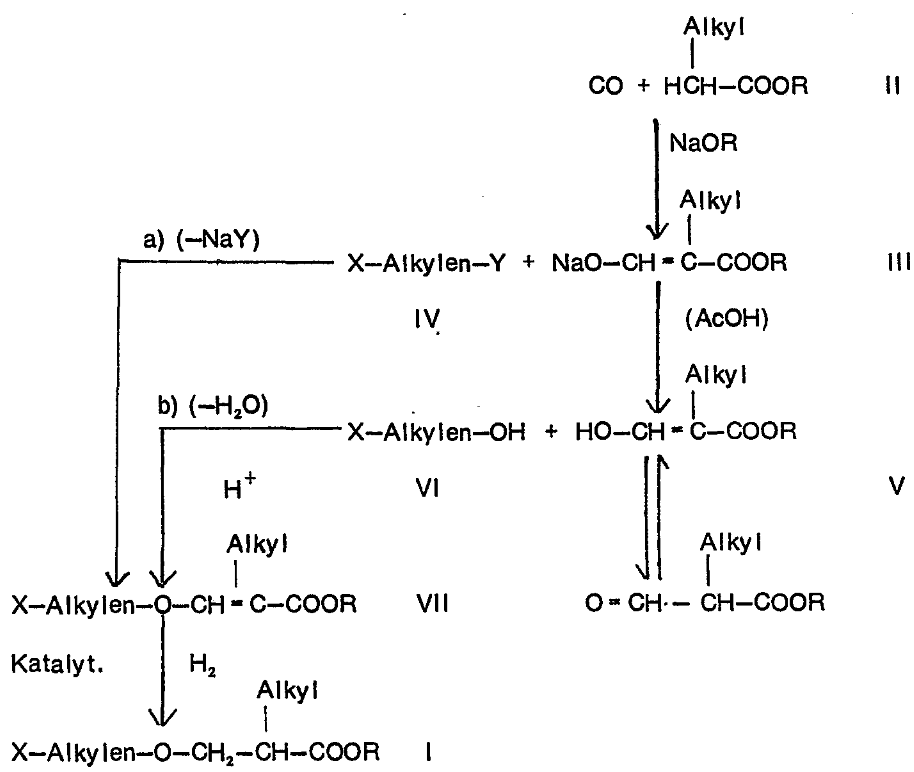


II, III, IV, V, VI, VII, I

Das bevorzugte Verfahren besteht darin, dass man das Alkalimetall-Enolat eines 2-Formylalkansäureesters der Formel (III) nach a) direkt mit einem Sulfonsäure-halogenalkylester der Formel (IV) zum entsprechenden 3-Halgenalkoxy-2-alkyl-acrylsäureester der Formel (VII) umsetzt und diesen anschliessend katalytisch hydriert zum gewünschten 3-Halogenalkoxy-2-alkylpropionsäureester der Formel (I).

Bevorzugte Verbindung im Hinblick auf die Verwendung zur Herstellung der auf Seite 1 erwähnten 2-[2-(3-Acetamino-2,4,6-trijod-phenoxy)-äthoxy]-methyl-buttersäure ist der 3-(2-Chloräthoxy)-2-äthyl-propionsäuremethylester, der am einfachsten erhalten wird durch Umsetzung des Natrium-Enolats von 2-Formylbuttersäuremethylester mit p-Toluolsulfonyloxyäthylchlorid und katalytische Hydrierung des dabei erhaltenen 3-(2-Chloräthoxy)-2-äthyl-acrylsäuremethylesters.

2-Formyl-alkansäureester der Formel (III) sind aus der Literatur bekannt. Sie werden beispielsweise nach Ingold et al [J.Chem.Soc. *121* (1922) 1782] oder Beer et al [deutsches Patent 708.513] erhalten durch Umsetzung von Alkansäureester der Formel (II) mit verhältnismässig teurem Ameisensäureester in Gegenwart eines basischen Kondensationsmittels wie z.B. Natrium oder Natriumalkoholat eventuell unter Anwendung von Ueberdruck.

Aus der französischen Patentanmeldung FR—A—2.128.410 ist auch bekannt, dass sich Alkansäureester mit Kohlenmonoxyd in Gegenwart von Natriummethylat formylieren lassen. Diese Formylierung gelingt bereits bei Anwendung eines geringen Ueberdruckes von Kohlenmonoxyd mit hervorragender Ausbeute.

Kondensationen von Oxo-Verbindungen mit Alkoholen zu den entsprechenden Enoläthern sind in der Literatur bereits beschrieben worden. Nach Gannon et al [Organic Syntheses *40*. (1960) 41] kann man beispielsweise Dihydroresorcin in Gegenwart von Toluolsulfonsäure mit Äthanol zu dem entsprechenden Enoläther, dem 3-Äthoxy-2-cyclohexenon, kondensieren.

Beschrieben ist auch die Umsetzung von Alkalimetall-Enolaten mit Alkylhalogeniden. Vergl. z.B. Houben-Weyl's Methoden der organischen Chemie *6/3*.109—110(1965). Enoläther sind im allgemeinen sehr labile Verbindungen, sie werden teilweise bereits durch Einwirkung von Wasser hydrolysiert. Houben-Weyls *7/1*.46 (1954).

Die Anwendung dieser schwer erkennbaren Analogien zur Herstellung von 3-Halogenalkoxy- oder 3-Hydroxyalkoxy-2-alkyl-acryl-säureestern lag demnach allerdings auch für den Fachmann nicht auf der Hand, umsomehr als er hätte annehmen müssen, dass dabei unhandliche, höchst labile, feuchtigkeitsempfindliche Produkte entstehen.

Schliesslich ist auch die Hydrierung eines Enoläthers vorbeschrieben worden. So haben Feely et al [Organic Syntheses *38* (1968) 22] durch katalytische Hochdruckhydrierung von Äthoxymethylenmalonsäurediäthylester und erhitzen des offenbar intermediär gebildeten 2-Äthoxymethyl-malonsäurediäthylesters den Methylenmalonsäurediäthylester erhalten.

Auch die Anwendung dieser Methode ist für den Fachmann zur Herstellung von 3-Halogenalkoxy-2-alkyl-propionsäureestern der Formel (I) nicht nahegelegen, unsomehr als er befürchten musste, dass unter den Bedingungen einer katalytischen Hochdruckhydrierung auch das Halogen-atom in den Verbindungen der allgemeinen Formel (V) und (I) durch Wasserstoff ersetzt wird. Ueberraschenderweise liessen sich die 3-Halogenalkoxy-2-alkyl-acrylsäureester der Formel (V) mit hervorragenden Ausbeuten zu den gewünschten 3-Halogenalkoxy-2-alkyl-propionsäureester der Formel (I) hydrieren. Ersatz von Halogen durch Wasserstoff tritt dabei nich auf.

Obschon Analogien zu einzelnen Elementen des vorliegenden neuen Verfahrens bekannt waren, stellt das Auffinden dieses überlegen einfachen, billigen und arbeitshygienischen Verfahrens einen Fortschritt dar.

## BEISPIELE:

### Beispiel 1:

### 3-(2-Chloräthoxy)-2-äthyl-propionsäuremethylester

1a: *2-Formyl-buttersäuremethylester*

130 g 85.2%iges Natriummethylat (2 Mol) werden im Autoklaven in mit 1200 ml Buttersäuremethylester (10,6 Mol) verrührt. Nun leitet man Kohlenmonoxyd in den Autoklaven bis ein Druck von ~11 bar erreicht ist. Man erwärmt während 6 Stunden auf 60°C, wobei man das verbrauchte Kohlenmonoxyd durch 4-maliges Aufpressen von frischem CO ersetzt. Nach dem Abkühlen versetzt man die gebildete Reaktionsmischung mit 500 g Eis und 150 ml Eisessig. Die organische leichtere Phase wird abgetrennt, getrocknet und im Vakuum fraktioniert destilliert.

Man erhält 229—234,3 g 2-Formyl-buttersäuremethylester vom Siedepunkt 72—70°C/66.5 mbar.

Ausbeute: 88—90% der Theorie bezogen auf eingesetztes Natriummethylat.

Der nicht verbrauchte Buttersäuremethylester wird im Destillationsvorlauf praktisch quantitativ zurückerhalten.

1b: *3-(2-Chloräthoxy)-2-äthyl-acrylsäuremethylester*

35 g 2-Formyl-buttersäuremethylester (0,27 Mol) werden in 250 ml Benzol gelöst, mit 26 g 2-Chloräthanol (0,324 Mol) und 700 mg Natriumhydrogensulfat versetzt und unter Rühren während 18 Stunden am Rückflusskühler gekocht, wobei das Reaktionswasser mit einem Separator abgetrennt wird. Das Lösungsmittel wird abdestilliert und der Rückstand wird im Vakuum fraktioniert.

Man erhält 42,8—44,2 g 3-(2-Chloräthoxy)-2-äthylacrylsäuremethylester vom Siedepunkt 99—101°C/4 mbar. $N_D^{23} = 1,4770$.

Ausbeute: 82,4—85% der Theorie.

Eine höher siedende Fraktion (Kp 102—130°/4 mbar) enthält grössere Mengen 3,3-Bis-(2-chloräthoxy)-2-äthyl-propionsäuremethylester. Durch Rückflusskochen in Benzol in Gegenwart von $NaHSO_4$ kann daraus 3-(2-Chloräthoxy)-2-äthyl-acrylsäureester erhalten werden.

1c: *3-(2-Chloräthoxy)-2-äthyl-propionsäuremethylester*

37 g 3-(2-Chloräthoxy)-2-äthyl-acrylsäuremethylester (0,192 Mol) werden in 350 ml Äthanol mit 6 g Raney-Nickel-Katalysator versetzt. Die Reaktionsmischung wird im Autoklaven bei 55—60°C und 30—60 bar $H_2$ hydriert. Der Katalysator wird abdestilliert und der Eindampfrückstand wird im Vakuum fraktioniert destilliert. Man erhält 30,7—33,7 g 3-(2-Chloräthoxy)-2-äthyl-propionsäuremethylester vom Siedepunkt 110°C/19 mbra. $N_D^{20} = 1,4399$, $d_4^{20} = 1,083$.

Ausbeute: 82—90% der Theorie.

Die selektive Reduktion der Doppelbindung gelingt mit gleichem Erfolg auch in Gegenwart von Palladium-Kohle-Katalysator (5% Pd) wenn man bei Normaldruck hydriert.

## Beispiel 2:
## 3-(2-Chloräthoxy)-2-äthyl-propionsäureäthylester

2a: *2-Formyl-buttersäureäthylester*

1200 g Buttersäureäthylester werden mit 130 g 85%igem Natriummethylat analog Beispiel 1a umgesetzt. Man erhält 245 g 2-Formylbuttersäureäthylester vom Siedepunkt 60—64°C/19 mbra. $n_{20}^D = 1,4300$.

Ausbeute: 85% der Theorie bezogen auf eingesetztes Natriummethylat (2 Mol).

2b: *3-(2-Chloräthoxy)-2-äthyl-acrylsäureäthylester*

28,8 g 2-Formyl-buttersäureäthylester werden in 250 ml Benzol mit 54 g 2-Chloräthanol in Gegenwart von 250 mg Natriumhydrogensulfat am Rückflusskühler gekocht, wobei entstandenes Reaktionswasser laufend abgetrennt wird. Analog beispiel 1b erhält man schliesslich 34,5 g 3-(2-Chloräthoxy)-2-äthyl-acrylsäureäthylester vom Siedepunkt 104—107°C/3 mbar. $n_D^{23} = 1,4719$.

Ausbeute: 84% der Theorie.

2c: *3-(2-Chloräthoxy)-2-äthyl-propionsäureäthylester*

37 g 3-(2-Chloräthoxy)-2-äthyl-acrylsäureäthylester (0,179 Mol) werden analog Beispiel 1c hydriert. Man erhält 30,5 g der Titelverbindung vom Siedepunkt 120—122°C/19 mbar, $n_D^{27} = 1,4352$.

Ausbeute: 81,5% der Theorie.

## Beispiel 3:
## 3-(2-Bromäthoxy)-2-äthyl-propionsäuremethylester

3a: *3-(2-Bromäthoxy)-2-äthyl-acrylsäuremethylester*

70 g 2-Formyl-buttersäuremethylester (0,54 Mol), 135 g 2-Bromäthanol (1,08 Mol), 500 ml Benzol und 1,4 g Natriumhydrogensulfat werden unter Rühren am Rückfluss gekocht. Entstehendes Reaktionswasser wird durch einen Dean-Stark-Separator laufend abgetrennt. Nach 15 Stunden sind insgesamt 12,5 ml wässerige Phase abgetrennt worden. Das Lösungsmittel wird abgedampft und der Rückstand in Vakuum fraktioniert destilliert. Man erhält 109 g 3-(2-Bromäthoxy)-2-äthyl-acrylsäuremethylester vom Siedepunkt 86—88°C/0,1 mbar. $n_D^{20} = 1,4971$.

Ausbeute: 85% der Theorie.

$C_8H_{13}BrO_3$ Br ber. 33,70%; gef. 33,47%.

3b: *3-(2-Bromäthoxy)-2-äthyl-propionsäuremethylester*

47,4 g 3-(2-Bromäthoxy)-2-äthyl-acrylsäuremethylester werden in 300 ml Methanol in Gegenwart von 8 g 5%igem feuchten Palladium-Kohle-Katalysator bei Raumtemperatur und bei Normaldruck hydriert. Nachdem ca. 75% der berechneten Menge Wasserstoff verbraucht sind, erwärmt man auf ca. 50°C, wonach auch der letzte Viertel des Wasserstoffs aufgenommen wird. Die Titration einer Probe der filtrierten Reaktionslösung mit Silbernitrat weist nach, dass nur ca. 1,1% des im Ausgangsmaterial vorhandenen Brom's als Brom-ionen vorliegen.

Das Lösungsmittel wird verdampft und der Rückstand im Vakuum destilliert. Man erhält 38,7 g 3-(2-Bromäthoxy)-2-äthyl-propionsäuremethylester. vom Siedepunkt 123—125°C/19 mbar; $n_D^{20} \doteq 1,4589$.

Ausbeute: 81% der Theorie.

$C_8H_{15}BrO_3$ Br ber. 33,41% gef. 33,48%.

Beispiel 4:

3-(2-Chloräthoxy)-2-äthyl-propionsäuremethylester

4a: *2-(p-Toluolsulfonyloxy)-äthyl-chlorid*

40 kg p-Toluolsulfochlorid werden bei −10°C in 60,4 kg Pyridin eingetragen. Unter Rühren lässt man innert 4 Stunden bei −5°C 14,5 kg Äthylenchlorhydrin (2-Chloräthanol) einlaufen. Das Reaktionsgemisch wird auf eine Mischung von 125 kg Wasser, 125 kg Eis und 60 kg Methylenchlorid gerührt. Man fügt bei max. 10°C Salzsäure 1:1 zu bis zum pH 3.

Die Methylenchlorid-Phase wird abgetrennt, mit Wasser und Natriumhydrogencarbonat-Lösung gewaschen, getrocknet und anschliessend vom Lösungsmittel befriet.

Man erhält 44,8 kg 2-(p-Toluolsulfonyloxy)-äthylchlorid.

Siedepunkt: 110—115°C/0,01 mbar.

Ausbeute: 100% der Theorie.

4b: *Natrium-Enolat von 2-Formylbuttersäureester*

1021 g Buttersäuremethylester [10 Mol], 225 g Natriummethylat (96%ig) [4 Mol] und 40 ml Methanol werden im Autoklaven gerührt. Man leitet nun Kohlenmonoxyd ein bis ein Druck von 60 bar erreicht ist. Man erwärmt nun auf 60—65°C und ersetzt das durch die eintretende Umsetzung verbrauchte Kohlenmonoxyd durch wiederholtes Aufpressen auf jeweils 40 bar. Nach etwa 2 Stunden ist die Umsetzung beendet. Der Autoklaveninhalt wird im Vakuum vollständig zur Trockene eingedampft, wobei aus dem Destillat unumgesetzter Buttersäuremethylester (529 g) zurückerhalten wird.

Der Eindampfrückstand 620 g besteht vorwiegend aus Natrium-Enolat von 2-Formyl-buttersäuremethylester.

4c: *3-(2-Chloräthoxy)-2-äthyl-acrylsäuremethylester*

Der Eindampfrückstand 620 g wird in 1,8 l Dimethylacetamid aufgeschlämmt, mit 845 g 2-(p-Toluolsulfonyloxy)-äthylchlorid versetzt und während 15—20 Stunden bei 30°C gerührt.

Die Reaktionsmischung wird in Eiswasser eingerührt durch Zusatz von Essigsäure auf pH 7 eingestellt. Der gebildete 3-Chloräthoxy-2-äthyl-acrylsäuremethylester wird mit Methylenchlorid extrahiert, das Extrakt wird mit Wasser gewaschen, getrocknet und eingeengt. Der Eindampfrückstand wird im Vakuum destilliert.

Man erhält 513 g 3-(2-Chloräthoxy-2-äthyl-acrylsäuremethylester vom Siedepunkt 72—75°C/0,1 mbar.

Ausbeuten: 74% der Theorie bezogen auf eingesetztes 2-p-Toluolsulfonyloxyäthylchlorid bzw.
67% der Theorie bezogen auf bei der Stufe 4b. eingesetztes Natriummethylat.

4d: *3-(2-Chloräthoxy)-2-äthyl-propionsäuremethylester*

346,8 g 3-(2-Chloräthoxy)-2-äthyl-acrylsäuremethylester in 1200 ml Methanol werden mit 27 g 5%igem Palladium-Kohler-Katalysator versetzt und bei Normaldruck hydriert. In etwa $1\frac{1}{2}$—2 Stunden wird die berechnete Menge Wasserstoff zur Absättigung der Doppelbindung aufgenommen. Der katalysator wird abfiltriert, das Filtrat wird eingedampft. Der Rückstand wird im Vakuum destilliert.

Man erhält 304 g 3-(2-Chloräthoxy)-2-äthyl-propionsäuremethylester vom Siedepunkt 110—112°C/19 mbar.

Ausbeute: 87% der Theorie.

Beispiel 5:

3-(2-Hydroxyäthoxy)-2-äthyl-propionsäuremethylester

5a: *3-(2-Hydroxyäthoxy)-2-äthyl-acrylsäuremethylester*

62 g nach Beispiel 4b hergestelltes Natrium-Enolat von 2-Formyl-buttersäuremethylester werden in 180 ml Dimethylacetamid aufgeschlämmt, mit 32 g 2-Chloräthanol versetzt und während 15—20 Stunden auf dem Wasserbad gerührt.

Die Aufarbeitung erfolgt analog wie im Beispiel 4c im einzelnen beschrieben wurde.

Man erhält 28,75 g 3-(2-Hydroxyäthoxy)-2-äthyl-acrylsäuremethylester vom Siedepunkt 110—116°C/0,1 mbar.

Ausbeute: 41% der Theorie.

$C_8H_{14}O_4$ C ber. 55,10% gef. 53,33%.

5b: *3-(2-Hydroxyäthoxy)-2-äthyl-propionsäuremethylester*

28 g 3-(2-Hydroxyäthoxy)-2-äthyl-acrylsäuremethylester in 100 ml Methanol werden mit 2 g 5%igem Palladium-Kohle-Katalysator versetzt und bei Normaldruck hydriert. Nachdem die berechnete Menge Wasserstoff verbraucht worden ist, wird der Katalysator abfiltriert, das Lösungsmittel abgedampft und das hinterbliebene rohe Produkt im Vakuum destilliert. Man erhält 24 g 3-(2-Hydroxyäthoxy)-2-äthyl-propionsäuremethylester vom Siedepunkt 104—107°C/0,1 mbar.

Ausbeute: 84% der Theorie.

$C_8H_{16}O_4$ C ber. 54,54% gef. 54,35%.

Beispiel 6:
3-(4-Chlorbutoxy)-2-butyl-propionsäuremethylester

6a: *Natrium-Enolat von 2-Formyl-capronsäuremethylester*

162,5 g Capronsäuremethylester [1,25 Mol], 28 g Natriummethylat (96%ig) [0,5 Mol] und 10 ml Methanol werden im Autoklaven analog dem im Beispiel 4b beschriebenen Verfahren mit Kohlenmonoxyd umgesetzt und aufgearbeitet.

Man erhält etwa 100 g Eindampfrückstand bestehend aus rohem Natrium-Enolat von 2-Formylcapronsäuremethylester.

6b: *3-(4-Chlorbutoxy)-2-butyl-acrylsäuremethylester*

Der Eindampfrückstand 6b. wird in 180 ml Dimethylacetamid suspendiert und mit 103 g 4-Chlorbutylbromid versetzt und während 4—6 Stunden bei 65—70°C gerührt. Die Reaktionsmischung wird analog wie im beispiel 5c beschrieben aufgearbeitet.

Man erhält 74,5 g 3-(4-Chlorbutoxy)-2-butyl-acrylsäuremethylester vom Siedepunkt 155°C/3 mbar.

Ausbeute: 60% der Theorie bezogen auf bei 6a. eingesetztes Natriummethylat.

6c: *3-(4-Chlorbutoxy)-2-butyl-propionsäuremethylester*

62,2 g (0,25 Mol) 3-(4-Chlorbutoxy)-2-butyl-acrylsäuremethylester wird analog Beispiel 4d. katalytisch hydriert. Man erhält 47 g 3-(4-Chlorbutoxy)-2-butylpropionsäuremethylester vom Siedepunkt 142°C/3 mbar.

Ausbeute: 75% der Theorie.

$C_{12}H_{23}Cl_3$ Cl ber. 14,14% gef. 14,30%.

Beispiel 7:
3-(2-Chloräthoxy)-2-methyl-propionsäurepropylester

7a: *3-(2-Chloräthoxy)-2-methyl-acrylsäurepropylester*

145 g Propionsäurepropylester [1,25 Mol], 28 g Natriummethylat (96%ig) [0,5 Mol] und 10 ml Methanol werden im Autoklaven analog Beispiel 4b. mit Kohlenmonoxyd umgesetzt und aufgearbeitet.

Der erhaltene Eindampfrückstand (92 g) wird analog Beispiel 4c. mit 141 g 2-(p-Toluolsulfonyloxy)-äthylchlorid bei 30°C umgesetzt und aufgearbeitet.

Man erhält 67,13 g 3-(2-Chloräthoxy-2-methyl-propionsäurepropylester vom Siedepunkt 97—99°C/1 mbar.

Ausbeute: 65% der Theorie bezogen auf eingesetztes Natriummethylat.

7b: *3-(2-Chloräthoxy)-2-methyl-propionsäurepropylester*

62 g 3-(2-Chloräthoxy)-2-methyl-acrylsäurepropylester (0,2 Mol) werden analog Beispiel 4d in Methanol bei Normaldruck katalytisch hydriert und aufgearbeitet.

Man erhält 55,7 g 3-(2-Chloräthoxy)-2-methyl-propionsäurepropylester vom Siedepunkt 102—105°C/7 mbar.

Ausbeute: 89% der Theorie.

$C_9H_{17}O_3Cl$ Cl ber. 17,0% gef. 16,8%.

**Patentansprüche**

1. Verfahren zur Herstellung eines 3-Halogenalkoxy-2-alkyl-propionsäureesters oder 3-Hydroxyalkoxy-2-alkyl-propionsäureesters der allgemeinen Formel

$$X\text{–Alkylen–O–}CH_2\text{–}\underset{\displaystyle\text{Alkyl}}{\overset{|}{CH}}\text{–COOR} \qquad \text{I,}$$

worin X Chlor, Brom, oder eine Hydroxy-Gruppe, Alkylen einen zweiwertigen Alkylen-Rest mit 2 bis 4 Kohlenstoffatomen, Alkyl einen Alkyl-Rest mit 1 bis 4 Kohlenstoffatomen und R einen Alkyl-Rest mit 1 bis 3 Kohlenstoffatomen darstellt, dadurch gekenzeichnet, dass man einen 2-Formylalkansäureester, der in an sich bekannter Weise durch Formylierung eines Alkansäureesters der allgemeinen Formel

$$\overset{\displaystyle\text{Alkyl}}{\underset{CH_2\text{–COOR}}{|}} \qquad \text{II,}$$

worin Alkyl einen Alkyl-Rest mit 1 bis 4 Kohlenstoffatomen und R einen Alkyl-Rest mit 1 bis 3 Kohlenstoffatomen darstellt, mit Kohlenmonoxyd unter geringem Ueberdruck in Gegenwart eines Alkalimetallalkoholates erhalten wird,

a) in der dabei unmittelbar gebildeten Form seines Alkalimetall-Enolates der Formel

$$\text{Alkalimetall}-O-CH=\underset{|}{\overset{\text{Alkyl}}{C}}-COOR \qquad \text{III},$$

direkt mit einem Halogen- oder Hydroxyalkylhalogenid oder mit einem Sulfonsäure-halogenalkyl- oder hydroxyalkylester der allgemeinen Formel

$$X-\text{Alkylen}-Y \qquad \text{IV},$$

worin X Chlor, Brom oder eine Hydroxy-Gruppe, Alkylen einen zweiwertigen Alkylen-Rest mit 2 bis 4 Kohlenstoffatomen und Y Chlor, Brom oder Jod, oder eine Aryl-oder Alkylsulfonyloxy-Gruppe mit 1 bis 7 Kohlenstoffatomen bedeutet, in einem nicht wässrigen basischen Milieu zur Reaktion bringt oder b) einen freigestzten 2-Formylalkansäureester der Formel

$$O=CH-\overset{\text{Alkyl}}{\overset{|}{CH}}-COOR \rightleftharpoons HO-CH=\overset{\text{Alkyl}}{\overset{|}{C}}-COOR \qquad \text{V},$$

mit einem Halogenalkanol oder Hydroxyalkanol der allgemeinen Formel

$$X-\text{Alkylen}-OH \qquad \text{VI},$$

worin X Chlor, Brom oder eine Hydroxy-Gruppe und Alkylen einen zweiwertigen Alkylen-Rest mit 2 bis 4 Kohlenstoffatomen bedeutet, in Gegenwart einer starken Säure und eines inerten organischen Lösungsmittels kondensiert und das Reaktionswasser laufend aus der Reaktionszone entfernt, wobei sowohl nach a) als auch nach b) der entsprechende 3-Halogenalkoxy- bzw. 3-Hydroxyalkoxy-2-alkyl-acrylsäureester der allgemeinen Formel

$$X-\text{Alkylen}-O-CH=\overset{\text{Alkyl}}{\overset{|}{C}}-COOR \qquad \text{VII},$$

entsteht und dass man diesen anschliessend durch Hydrierung in den gewünschten 3-Halogenalkoxy- bzw. 3-Hydroxyalkoxy-2-alkyl-propionsäureester überführt.

2. Verfahren nach Patentanspruch 1, dadurch gekennzeichnet, dass man das Alkalimetall-Enolat eines 2-Formylalkansäureesters der Formel (III) nach a) direkt mit einem Sulfonsäure-halogenalkylester der Formel (IV) zum entsprechenden 3-Halogenalkoxy-2-alkyl-acrylsäureester der Formel (VII) umsetzt und diesen anschliessend katalytisch hydriert zum gewünschten 3-Halogenalkoxy-2-alkyl-propionsäureester der Formel (I).

3. Verfahren nach Patentanspruch 1, dadurch gekennzeichnet, dass man das Natrium-Enolat von 2-Formyl-buttersäuremethylester mit einem 2-Aryl- oder 2-Alkylsulfonyloxyäthylchlorid, vorzugsweise mit 2-p-Toluolsulfonyloxyäthylchlorid, zum 3-(2-Chloräthoxy)-2-äthyl-acrylsäuremethylester umsetzt und diesen durch katalytische Hydrierung in den gewünschten 3-(2-Chloräthoxy)-2-äthyl-propionsäuremethylester überführt.

**Revendications**

1. Procédé pour la préparation d'un ester 3-halogénoalcoxy-2-alkyl-propionique ou d'un ester 3-hydroxyalcoxy-2-alkyl-propionique répondant à la formule générale

$$X-\text{Alkylèn}-O-CH_2-\overset{\text{Alkyl}}{\overset{|}{CH}}-COOR \qquad \text{I},$$

dans laquelle X représente le chlore, le brome ou un groupe hydroxy, "Alkylèn" représente un radical alkylène divalent contenant de 2 à 4 atomes de carbone, "Alkyl" représente un radical alkyle contenant de 1 à 4 atomes de carbone, et R un radical alkyle contenant de 1 à 3 atomes de carbone, caractérisé en ce que l'on part d'un ester 2-formyl-alcanoïque obtenu de manière connue en soi par formylation d'un ester alcanoïque répondant à la formule générale

$$\overset{\text{Alkyl}}{\overset{|}{CH_2}}-COOR \qquad \text{II},$$

dans laquelle "Alkyl" représente un radical alkyle contenant de 1 à 4 atomes de carbone, et R un radical alkyle contenant de 1 à 3 atomes de carbone, à l'aide d'oxyde de carbone, sous légère surpression, en présence d'un alcoolate de métal alcalin, et

a) on le fait réagir, sous la forme sous laquelle il a été obtenu directement, d'énolate de métal alcalin répondant à la formule

Alkyl
|
Métal alcalin–O–CH=C–COOR III,

avec un halogénure d'halogénoalkyle ou d'hydroxyalkyle ou avec un ester halogénoalkylique ou hydroxyalkylique d'acide sulfonique répondant à la formule générale

X–Alkylèn–Y IV,

dans laquelle X représente le chlore, le brome ou un groupe hydroxy, "Alkylèn" représente un radical alkylène divalent contenant de 2 à 4 atomes de carbone et Y représente le chlore, le brome ou l'iode, ou bien un groupe arylsulfonyloxy ou alkylsulfonyloxy contenant de 1 à 7 atomes de carbone, dans un milieu basique non aqueux, ou bien

b) on condense un ester 2-formylalcanoïque libéré de formule

Alkyl Alkyl
| |
O=CH–CH–COOR ⇌ HO–CH=C–COOR V,

avec un halogénoalcanol ou hydroxyalcanol de formule générale

X–Alkylèn–OH VI,

dans laquelle X représente le chlore, le brome ou un groupe hydroxy et "Alkylèn" représente un radical alkylène divalent contenant de 2 à 4 atomes de carbone, en présence d'un acide fort et d'un solvant organique inerte en éliminant en continu de la zone de réaction l'eau formée dans la réaction, ces opérations donnant, dans le cas a) comme dans le cas b), l'ester 3-halogénoalcoxy- ou respectivement 3-hydroxyalcoxy-2-alkyl-acrylique correspondant qui répond à la formule générale

Alkyl
|
X–Alkylèn–O–CH=C–COOR VII,

qu'on convertit ensuite par hydrogénation en l'ester 3-halogénoalcoxy- ou respectivement 3-hydroxy-alcoxy-2-alkyl-propionique recherché.

2. Procédé selon la revendication 1, caractérisé en ce que l'on fait réagir directement l'énolate de métal alcalin d'un ester 2-formylalcanoïque de formule III, selon le mode opératoire a), avec un ester halogénoalkylique d'acide sulfonique de formule IV, formant ainsi l'ester 3-halogénoalcoxy-2-alkyl-acrylique correspondant de formule VII qu'on convertit finalement par hydrogénation catalytique en l'ester 3-halogénoalcoxy-2-alkyl-propionique recherché de formule I.

3. Procédé selon la revendication 1, caractérisé en ce que l'on fait réagir l'énolate de sodium du 2-formyl-butyrate de méthyle avec un chlorure de 2-aryl- ou 2-alkyl-sulfonyloxyéthyle, de préférence avec le chlorure de 2-p-toluène-sulfonyloxyéthyle, formant ainsi le 3-(2-chloréthoxy)-2-éthyl-acrylate de méthyle qu'on convertit par hydrogénation catalytique en le 3-(2-chloréthoxy)-2-éthylpropionate de méthyle recherché.

**Claims**

1. Process for the manufacture of a 3-halogenalkoxy-2-alkyl-propionic acid ester or 3-hydroxy-alkoxy-2-alkyl-propionic acid ester of the general formula

alkyl
|
X–alkylen–O–$CH_2$–CH–COOR I,

wherein X represents chlorine, bromine or a hydroxyl group, alkylene represents a divalent alkylene radical having 2 to 4 carbon atoms, alkyl represents an alkyl radical having 1 to 4 carbon atoms and R represents an alkyl radical having 1 to 3 carbon atoms, characterised in that a 2-formylalkanoic acid ester which is obtained in a manner known per se by formylation of an alkanoic acid ester of the general formula

alkyl
|
$CH_2$–COOR II,

wherein alkyl represents an alkyl radical having 1 to 4 carbon atoms and R represents an alkyl radical having 1 to 3 carbon atoms, with carbon monoxide under a slightly elevated pressure in the presence of an alkali metal alcoholate,

a) is reacted in the thus immediately resulting form of its alkali metal enolate of the formula

alkyl
|
alkali metall–O–CH=C–COOR III.

in a non-aqueous basic medium directly with a halogenoalkyl halide or hydroxyalkyl halide or with a sulphonic acid halogenoalkyl ester or hydroxyalkyl ester of the general formula

X–alkylene–Y IV,

wherein X represents chlorine, bromine or a hydroxyl group, alkylene represents a divalent alkylene radical having 2 to 4 carbon atoms and Y represents chlorine, bromine or iodine, or an arylsulphonyloxy group or alkylsulphonyloxy group having 1 to 7 carbon atoms, or
b) a liberated 2-formylalkanoic acid ester of the formula

alkyl alkyl
| |
O=CH–CH–COOR $\rightleftharpoons$ HO–CH=C–COOR V,

is subjected in the presence of a strong acid and of an inert organic solvent to a condensation reaction with a halogenoalkanol or a hydroxyalkanol of the general formula

X–alkylene–OH VI,

wherein X represents chlorine, bromine or a hydroxyl group and alkylene represents a divalent alkylene radical having 2 to 4 carbon atoms, and the water of reaction is continuously removed from the reaction zone, the corresponding 3-halogenoalkoxy- or 3-hydroxyalkoxy-2-alkylacrylic acid ester of the general formula

alkyl
|
X–alkylene–O–CH=C–COOR VII,

being formed both according to a) and according to b), and that this ester is subsequently converted by hydrogenation into the desired 3-halogenalkoxy- or 3-hydroxyalkoxy-2-alkyl-propionic acid ester.

2. Process according to Patent Claim 1, characterised in that the alkali metal enolate of a 2-formyl-alkanoic acid ester of the formula (III) is reacted according to a) directly with a sulphonic acid halogenoalkyl ester of the formula (IV) to give the corresponding 3-halogenoalkoxy-2-alkyl-acryl acid ester of the formula (VII) and the latter is subsequently hydrogenated catalytically to give the desired 3-halogenoalkoxy-2-alkyl-propionic acid ester of the formula (I).

3. Process according to Patent Claim 1, characterised in that the sodium enolate of 2-formyl-butyric acid methyl ester is reacted with a 2-aryl- or 2-alkyl-sulphonyloxyethyl chloride, preferably with 2-p-toluenesulphonyloxyethyl chloride, to give 3-(2-chloroethoxy)-2-ethyl-acrylic acid methyl ester and the latter is converted by catalytic hydrogenation into the desired 3-(2-chloroethoxy)-2-ethyl-propionic acid methyl ester.